# EUROPÄISCHE PATENTSCHRIFT

(11) **EP 0 520 344 B1**
(45) Veröffentlichungstag und Bekanntmachung des Hinweises auf die Patenterteilung: **28.02.1996**
(21) Anmeldenummer: 92110436.0
(22) Anmeldetag: 20.06.1992
(51) Int. Cl.: A43B 7/38, A61F 3/00

(54) **Gehhilfe**
Walking aid
Accessoire d'aide à la marche

(30) Priorität: 22.06.1991 DE 4120648; 12.12.1991 DE 4140914
(43) Veröffentlichungstag der Anmeldung: 30.12.1992
(73) Patentinhaber: BLÖMER, Alois, D-45964 Gladbeck (DE)
(72) Erfinder: Blömer, Alois, W-4390 Gladbeck (DE); Höhner, Peter, W-2844 Stemshorn (DE); Schriever, Hartmut, W-2840 Diepholz (DE); Adler, Lothar, W-4500 Osnabrück (DE)
(74) Vertreter: Spalthoff, Adolf, Dipl.-Ing.

(56) Entgegenhaltungen:
- US-A- 3 758 891
- US-A- 3 919 789
- US-A- 4 265 033

## Beschreibung

Die Erfindung bezieht sich auf eine Gehhilfe bestehend aus einem an einem verletzten Bein einer Person anbringbaren Gehstollen für einen der Ruhigstellung und/oder der Stützung dienenden Unter- und Oberschenkelverband.

Bei der Anwendung derartige Gehstollen aufweisender Gehhilfen ergibt sich, daß infolge des das verletzte Bein fixierenden Verbands und des Gehstollens das kranke Bein gegenüber dem gesunden Bein zumindest um die Stärke des Gehstollens höher angeordnet ist.

Dies bringt als Nachteil ein biologisch ungünstiges Verhalten für das kranke Bein mit sich, zumal das gesamte Körpergewicht der verletzten Person mittels des kranken Beins über das Niveau des gesunden Beins angehoben werden muß, mit der Folge einer raschen Ermüdung sowie sich ausbildender Rückenschmerzen.

Der Erfindung liegt die Aufgabe zugrunde, eine Gehhilfe zu schaffen, bei der die vorstehend erwähnten nachteiligen Wirkungen nicht auftreten.

Diese Aufgabe wird erfindungsgemäß dadurch gelöst, daß die Gehhilfe weiter eine Ausgleichsohle aufweist, die unter dem Schuh des gehstollenfreien gesunden Beins einer Person anbringbar ist. Von dort kann sie später wieder entfernt werden. Hierdurch ergibt sich eine wesentliche Entlastung des kranken Beins, wodurch einer raschen Ermüdung sowie einer etwaigen Fehlbelastung mit sich daraus ergebenden Rückenschmerzen entgegengewirkt wird.

Sofern die Dicke der Ausgleichsohle derjenigen Höhendifferenz entspricht, die sich aufgrund des Gehstollens für das verletzte Bein ergibt, können die vorstehend aufgeführten nachteiligen Wirkungen nicht nur reduziert sondern völlig beseitigt werden.

In konstruktiv einfacher Weise kann die Ausgleichsohle so gestaltet werden, daß sie sich über die gesamte Länge des am gesunden Bein sich befindlichen Schuhs erstreckt.

Es ist jedoch auch möglich, die Ausgleichsohle mittels einer Kerbe in einen Sohlenabschnitt und ein Absatzstück trennbar zu gestalten, womit sichergestellt ist, daß die Laufsohle ausschließlich an denjenigen Flächenbereichen der Schuhsohle sich befindet, die beim Gehen mit dem Untergrund in Berührung kommen. Durch diese Aufteilung der Ausgleichsohle in Sohlenabschnitt und Absatzstück läßt sich außer einer Materialeinsparung an dem für die Herstellung der Ausgleichsohle verwendeten hochwertigen Elastomer-Werkstoff eine starke Flexibilität der Ausgleichsohle erreichen.

Wenn die Ausgleichsohle an ihrer Unterseite mittels Nuten und Rippen profiliert ist, ergibt sich eine weitere Erhöhung der Elastizität und Flexibilität, wobei zusätzlich eine gewisse Sicherheit gegen Rutschen erreicht werden kann. Darüber hinaus wird durch diese Ausgestaltung die Biegsamkeit der Ausgleichsohle erhöht.

Um die Elastizität der Ausgleichsohle weiter zu erhöhen, können in der ersten Nut bzw. Rippe oder auch in weiteren Nuten bzw. Rippen zusätzliche Quereinschnitte vorhanden sein, so daß auch eine bessere Anpassung der Ausgleichsohle an die Schuhsohle gegeben ist.

Sofern das zur Herstellung der Ausgleichsohle verwendete Material elastisch ist, ergibt sich ein nachgiebiger Gang, womit eine Reduzierung der Belastungen für die verletzte Person erreicht wird.

In einfacher Weise läßt sich die Anbringung der Ausgleichsohle am Schuh bewerkstelligen, wenn die Oberseite der Ausgleichsohle an die Unterseite des Schuhs klebbar ist.

Eine solche Klebverbindung läßt sich besonders einfach verwirklichen, wenn eine ausgleichsohlenseitige Klebfläche selbstklebend ausgebildet und mit einer Abreißhilfe versehen ist.

Sofern die Klebverbindung zwischen Ausgleichsohle und Schuh nach Beendigung der Nutzungsdauer der Ausgleichsohle lösbar ist, kann nach Beendigung der ärztlichen Behandlung die Ausgleichsohle von dem normalen Schuhwerk wieder entfernt werden.

Besonders günstige Dämpfungs- und Federungseigenschaften ergeben sich für die Ausgleichsohle, wenn diese aus dem Material "Cellasto" (WZ d. Fa. BASF) hergestellt wird, bei dem es sich um einen federnden und dämpfenden Werkstoff aus Polyurethan-Elastomer handelt.

Eine leichte Anpaßbarkeit der Ausgleichsohle läßt sich erreichen, wenn diese einen in ihrer Längsrichtung verlaufenden Schlitz aufweist. Dann kann die Ausgleichsohle vor ihrem Ankleben an die Schuhsohle auseinandergezogen werden, bis die Breite der Ausgleichsohle der Breite der Schuhsohle entspricht. Auf diese Art und Weise ergibt sich eine Ausgestaltung der Ausgleichsohle, bei der sie hinsichtlich ihrer Breite in einfachster Weise an die Breite des Schuhs anpaßbar ist.

Sofern die Ausgleichsohle mehrschichtig ausgebildet ist, wobei die in Vertikalrichtung untereinander angeordneten Schichten miteinander verklebbar sind, kann die Ausgleichsohle durch Ergänzen oder Weglassen von Schichten in besonders leichter Weise in der für den angestrebten Zweck erforderlichen Dicke ausgebildet werden. Hierbei kann die Anpassung der Ausgleichsohle an den Unter- und Oberschenkelverband nach dessen Herstellung unmittelbar am Patienten erfolgen.

Sofern die Schichten der Ausgleichsohle in unterschiedlichen Dicken vorliegen, ergibt sich eine noch bessere Anpaßbarkeit der vertikalen Dicke der Ausgleichsohle an den aktuellen Anwendungsfall.

Wenn die einzelnen Schichten der Ausgleichsohle an ihren Klebflächen Vorsprünge bzw. Ausnehmungen aufweisen, die zwecks Positionierung der Schichten aneinander miteinander in Eingriff bringbar sind, können die einzelnen Schichten der Ausgleichsohle in wenig aufwendiger Weise durch einfache Handgriffe genau in bezug aufeinander positioniert werden.

Es ist nicht zwingend erforderlich, daß die Ausgleichsohle in Verbindung mit einem aus Laufsohle und Stützverband bestehenden Gehstollen Anwendung findet. Es ist auch denkbar, daß eine derartige Ausgleichsohle für orthopädische Schuhe Verwendung findet, um eine Entlastung der Wirbelsäule zu erreichen, was insbesondere erreicht wird, wenn für die Ausgleichsohle ein Werkstoff mit guten dämpfenden Eigenschaften gewählt wird. Für diesen Anwendungsfall eignet sich eine Fertigung der Ausgleichsohle vom Band, so daß die benötigte Größe jeweils abgelängt werden kann.

Die US-A-3 758 891 beschreibt eine in unterschiedlicher Weise mit einem Fuß verbindbare Sohle, die zu unterschiedlichen

Im folgenden wird die Erfindung an Hand einer Ausführungsform unter Bezugnahme auf die Zeichnung näher erläutert.

Es zeigen:
- Figur 1: eine Draufsicht auf eine Auführungsform der erfindungsgemäßen Ausgleichsohle;
- Figur 2: einen Schnitt längs der Linie II - II in Figur 1;
- Figur 3: eine Seitenansicht der obersten Schicht einer dreischichtigen Ausführungsform der erfindungsgemäßen Ausgleichsohle;
- Figur 4: die mittlere Schicht der dreischichtigen Ausführungsform der erfindungsgemäßen Ausgleichsohle;
- Figur 5: die unterste Schicht der dreischichtigen Ausführungsform der erfindungsgemäßen Ausgleichsohle; und
- Figur 6: die Ansicht einer Klebefläche einer der drei Schichten der Ausgleichsohle gemäß den Figuren 3 bis 5.

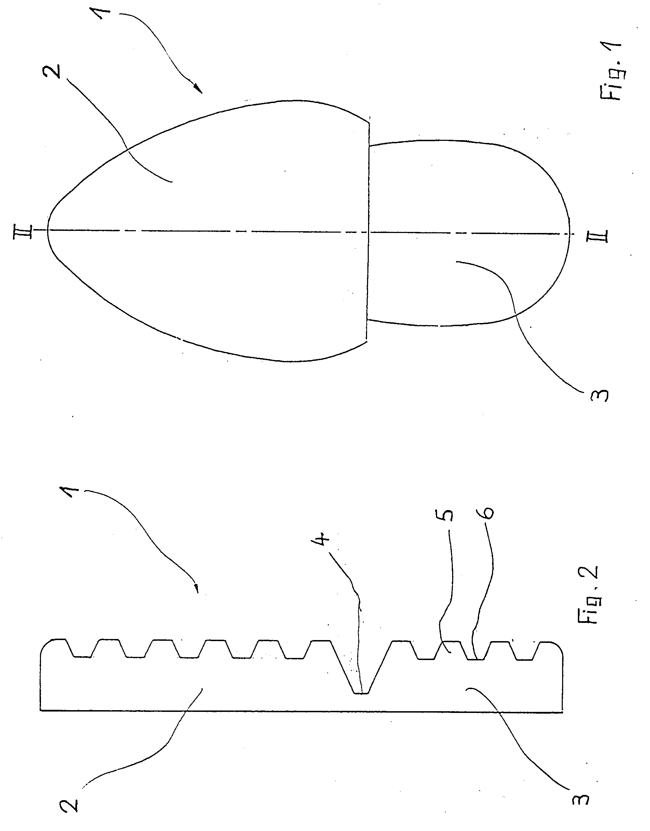

Eine in den Figuren 1 und 2 dargestellte Ausgleichsohle 1 wird unterhalb der Sohle des am gehstollenfreien Bein getragenen Schuhs angebracht, um die sich durch die Verwendung des Gehstollens am verletzten Bein ergebende Höhendifferenz zwischen den beiden Beinen auszugleichen.

Die Ausgleichsohle 1 hat einen Sohlenabschnitt 2 und ein Absatzstück 3. Der Sohlenabschnitt 2 wird an demjenigen Bereich der Schuhsohle angebracht, der beim Gehen mit dem Boden in Berührung kommt. Das Absatzstück 3 wird am Schuhabsatz angebracht.

Zwischen dem Sohlenabschnitt 2 und dem Absatzstück 3 ist eine Kerbe 4 ausgebildet, an der die Ausgleichsohle 1 in ihren Sohlenabschnitt 2 und ihr Absatzstück 3 aufteilbar ist. Diese Aufteilung kann durch Schneiden, Sägen, Abscheren od.dgl. geschehen.

Als Werkstoff für die Ausgleichsohle 1 wird ein elastisches Material verwendet. Zur Verbesserung der Elastizitätseigenschaft ist die Ausgleichsohle 1 an ihrer die Lauffläche bildenden Unterseite mit Rippen 5 und Nuten 6 ausgestaltet. Diese können auch die Griffeigenschaften der Ausgleichsohle 1 verbessern, was insbesondere bei glatten Untergründen bedeutsam ist.

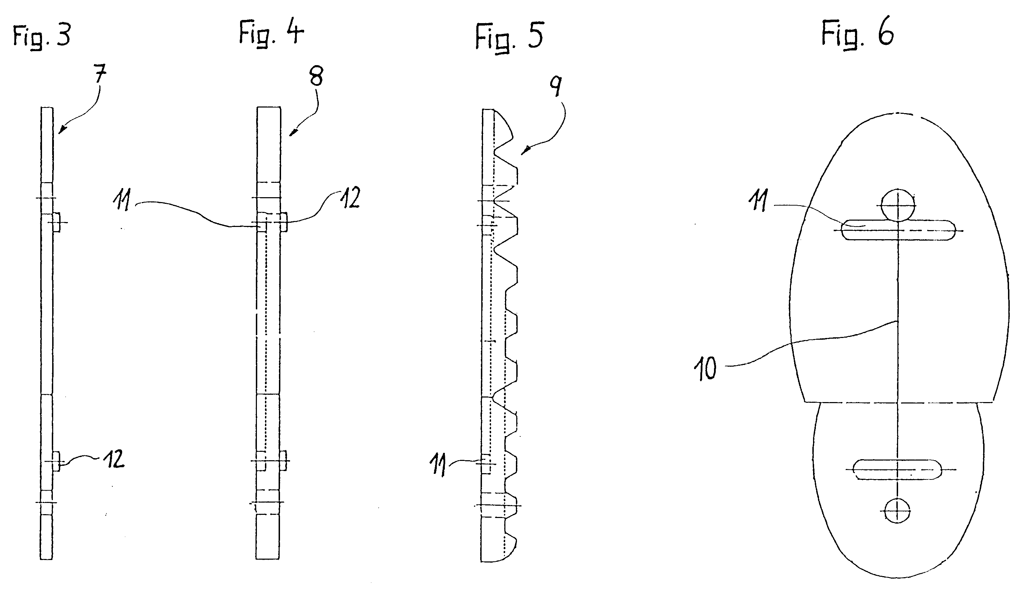

In den Figuren 3 bis 6 ist eine Ausführungsform der Ausgleichsohle 1 dargestellt, die einen dreischichtigen Aufbau aufweist, Die Figuren 3 bis 5 zeigen die oberste Schicht 7, die Mittelschicht 8 sowie die unterste Schicht 9 der Ausgleichsohle 1. In Figur 6 ist eine Klebefläche der Schichten 7, 8, 9 dargestellt. Jede Schicht 7, 8, 9 weist einen Längsschlitz 10 auf, der in Dickenrichtung die gesamte Schicht 7, 8, 9 durchdringt. Beim Anbringen der Schichten 7, 8, 9 kann diese durch Verbreiterung des Schlitzes 10 hinsichtlich ihrer Breite an diejenige der Schuhsohle angepaßt werden.

Des weiteren sind an den Klebflächen der Schichten 7, 8, 9 Vorsprünge 12 bzw. Ausnehmungen 11 ausgebildet. Beim Zusammenlegen der Schichten 7, 8, 9 werden die Vorsprünge 12 in die betreffenden Ausnehmungen 11 eingeführt, so daß eine korrekte Positionierung der Schichten aneinander zur Ausbildung der mehrschichtigen Ausgleichsohle 1 in einfacher Weise möglich ist.

## Patentansprüche

1. Gehhilfe bestehend aus einem an einem verletzten Bein einer Person anbringbaren Gehstollen für einen der Ruhigstellung und/oder Stützung dienenden Unter- und Oberschenkelverband, dadurch gekennzeichnet, daß die Gehhilfe weiter eine Ausgleichsohle (1) aufweist, die unter dem Schuh des gehstollenfreien Beins der Person anbringbar ist.

2. Gehhilfe nach Anspruch 1, bei der die Dicke der Ausgleichsohle (1) derjenigen Höhendifferenz entspricht, die aufgrund des Gehstollens bzw. des Verbands für das verletzte Bein auftritt.

3. Gehhilfe nach Anspruch 1 oder 2, bei der sich die Ausgleichsohle (1) über die gesamte Länge des Schuhs erstreckt.

4. Gehhilfe nach Anspruch 1 oder 2, bei der die Ausgleichsohle (1) an einer Kerbe (4) in einen Sohlenabschnitt (2) und ein Absatzstück (3) trennbar ist.

5. Gehhilfe nach einem der Ansprüche 1 - 4, bei der die Ausgleichsohle (1) an ihrer Unterseite mittels Nuten (6) und Rippen (5) profiliert ist.

6. Gehhilfe nach Anspruch 5, bei der in der ersten Nut (6) bzw. Rippe (5) oder auch in weiteren Nuten (6) bzw. Rippen (5) Quereinschnitte ausgebildet sind.

7. Gehhilfe nach einem der Ansprüche 1 - 6, bei der der zur Herstellung der Ausgleichsohle (1) verwendete Werkstoff elastisch ist.

8. Gehhilfe nach einem der Ansprüche 1 - 7, bei der zur Anbringung der Ausgleichsohle (1) am Schuh die Oberseite der Ausgleichsohle (1) an die Unterseite des Schuhs klebbar ist.

9. Gehhilfe nach Anspruch 8, bei der eine ausgleichsohlenseitige Klebfläche selbstklebend ausgebildet und mit einer Abreißhilfe versehen ist.

10. Gehhilfe nach Anspruch 8 oder 9, bei der die Klebverbindung zwischen Ausgleichsohle (1) und Schuh nach Beendigung der Nutzungsdauer der Ausgleichsohle (1) lösbar ist.

11. Gehhilfe nach einem der Ansprüche 1 - 10, bei der die Ausgleichsohle (1) aus einem federnden und dämpfenden Elastomer-Werkstoff, vorzugsweise aus Polyurethan-Elastomer, besteht.

12. Gehhilfe nach einem der Ansprüche 1 - 11, bei der die Ausgleichsohle (1) einen in ihrer Längsrichtung verlaufenden Schlitz (10) aufweist, so daß sie hinsichtlich ihrer Breite an den Schuh anpaßbar ist.

13. Gehhilfe nach einem der Ansprüche 1 - 12, bei der die Ausgleichsohle (1) mehrschichtig ausgebildet ist, wobei die in Vertikalrichtung untereinander angeordneten Schichten (7, 8, 9) miteinander verklebbar sind.

14. Gehhilfe nach Anspruch 13, bei der die Schichten (7, 8, 9) unterschiedliche Dicken aufweisen.

15. Gehhilfe nach Anspruch 13 oder 14, bei der die Schichten (7, 8, 9) an ihren Klebflächen Vorsprünge (12) bzw. Ausnehmungen (11) aufweisen, die zwecks Positionierung der Schichten (7, 8, 9) aneinander miteinander in Eingriff bringbar sind.

## Claims

1. Mobility aid, consisting of a mobility support which can be attached to an injured leg belonging to a person and which is for an upper leg and lower leg bandage, the purpose of which bandage is immobilization and/or support, characterised in that the mobility aid further comprises a compensation sole (1) which can be attached under the shoe of the leg of the person which is without the mobility aid.

2. Mobility aid according to Claim 1, in the case of which the thickness of the compensation sole (1) corresponds to the height difference which occurs as a result of the mobility support or the bandage for the injured leg.

3. Mobility aid according to Claim 1 or Claim 2, in the case of which the compensation sole (1) extends over the entire length of the shoe.

4. Mobility aid according to Claim 1 or Claim 2, in the case of which the compensation sole (1) can be separated into a sole section (2) and a heel piece (3) at a notch (4).

5. Mobility aid according to one of Claims 1 to 4, in the case of which the compensation sole (1) is profiled at its lower side by means of grooves (6) and ribs (5).

6. Mobility aid according to Claim 5, in the case of which transverse grooves are formed in the first groove (6) or rib (5) or even in further grooves (6) or ribs (5).

7. Mobility aid according to one of Claims 1 to 6, in the case of which the material used for the production of the compensation sole (1) is resilient.

8. Mobility aid according to one of Claims 1 to 7, in the case of which the upper side of the compensation sole (1) can be stuck to the underside of the shoe to attach the compensation sole (1).

9. Mobility aid according to Claim 8, in the case of which an adhesive surface is formed on the compensation sole side in a self-adhesive manner and is provided with a tear-off aid.

10. Mobility aid according to Claim 8 or Claim 9, in the case of which the adhesive connection between the compensation sole (10) and the shoe can be detached when the compensation sole (1) is no longer required.

11. Mobility aid according to one of Claims 1 to 10, in the case of which the compensation sole (1) consists of a resilient and muffling elastomer material, preferably polyurethane elastomer.

12. Mobility aid according to one of Claims 1 to 11, in the case of which the compensation sole (1) comprises a slot (10) which extends in the longitudinal direction thereof, such that it can be adapted to the shoe as regards its width.

13. Mobility aid according to any one of Claims 1 to 12, in the case of which the compensation sole (1) is formed with several layers, wherein the layers (7, 8, 9), which are arranged one below another in the vertical direction, can be adhered to one another.

14. Mobility aid according to Claim 13, in the case of which the layers (7, 8, 9) have different thicknesses.

15. Mobility aid according to Claim 13 or Claim 14, in the case of which the layers (7, 8, 9) comprise, on their adhesive surfaces, projections (12) or recesses (11) which can be brought into engagement with one another for the purpose of positioning the layers (7, 8, 9).

## Revendications

1. Appareil d'aide à la marche constitué d'un appui de marche adaptable sur la jambe blessée d'une personne dans un appareil prenant la cuisse et la jambe assurant l'immobilisation et/ou servant de support, caractérisé par le fait l'appareil d'aide à la marche comprend en outre une semelle (1) de compensation qui peut être appliquée sous la chaussure de la jambe ne comportant pas d'appui de marche.

2. Appareil d'aide à la marche selon la revendication 1, dans lequel l'épaisseur de la semelle de compensation (1) correspond à la différence de hauteur engendrée par l'appui de marche ou par l'appareil-pour la jambe blessée.

3. Appareil d'aide à la marche selon la revendication 1 ou la revendication 2, dans lequel la semelle de compensation (1) s'étend sur la totalité de la longueur de la chaussure.

4. Appareil d'aide à la marche selon la revendication 1 ou la revendication 2, dans lequel la semelle de compensation (1) peut être séparée en une partie semelle (2) et un partie talon (3) au niveau d'une entaille (4).

5. Appareil d'aide à la marche selon l'une des revendications 1 à 4, dans lequel la semelle de compensation (1) est pourvue sur sa face inférieure de profils formés de rainures (6) et de nervures (5).

6. Appareil d'aide à la marche selon la revendication 5, dans lequel des entailles transversales sont formées dans la première rainure (6) ou nervure (5) ou dans d'autres rainures (6) ou nervures (5).

7. Appareil d'aide à la marche selon l'une des revendications 1 à 6, dans lequel le matériau utilisé pour fabriquer la semelle de compensation (1) est élastique.

8. Appareil d'aide à la marche selon l'une des revendications 1 à 7, dans lequel la face supérieure de la semelle de compensation (1) peut être collée sur la face inférieure de la chaussure aux fins de fixer ladite semelle de compensation (1) sur la chaussure.

9. Appareil d'aide à la marche selon la revendication 8, dans lequel une surface de collage de la semelle de compensation est auto-adhésive et est pourvue d'un moyen d'aide au décollage.

10. Appareil d'aide à la marche selon la revendication 8 ou 9, dans lequel la liaison collée entre la semelle de compensation (1) et la chaussure peut être annulée à la fin de durée d'utilisation de ladite semelle de compensation (1).

11. Appareil d'aide à la marche selon l'une des revendications 1 à 10, dans lequel la semelle de compensation (1) est réalisée en matériau élastomère élastique et amortisseur, de préférence en un élastomère à base de polyuréthane.

12. Appareil d'aide à la marche selon l'une des revendications 1 à 11, dans lequel la semelle de compensation (1) comporte une fente (10) qui s'étend dans sa direction longitudinale de telle sorte que la largeur de ladite semelle peut être adaptée à la largeur de la chaussure.

13. Appareil d'aide à la marche selon l'une des revendications 1 à 12, dans lequel la semelle de compensation (1) comporte plusieurs couches, les différentes couches (7, 8, 9) disposées les unes sur les autres dans la direction verticale pouvant être collées entre elles.

14. Appareil d'aide à la marche selon la revendication 13, dans lequel les couches (7, 8, 9) ont des épaisseurs différentes.

15. Appareil d'aide à la marche selon la revendications 13 ou la revendication 14, dans lequel les couches (7, 8, 9) présentent sur leurs surfaces de collage des saillies (12) et des évidements qui peuvent être amenés en prise mutuelle à des fins de positionnement des couches (7, 8, 9).
